# EUROPÄISCHE PATENTSCHRIFT

(11) **EP 1 684 652 B1**
(45) Veröffentlichungstag und Bekanntmachung des Hinweises auf die Patenterteilung: **26.03.2008**
(21) Anmeldenummer: 04797690.7
(22) Anmeldetag: 06.11.2004
(51) Int. Cl.: A61B 17/86, A61B 17/70, A61B 17/64

(54) **KNOCHENVERANKERUNGSELEMENT UND STABILISIERUNGSEINRICHTUNG MIT EINEM DERARTIGEN KNOCHENVERANKERUNGSELEMENT**
BONE FIXING ELEMENT AND STABILISING DEVICE COMPRISING ONE SUCH BONE FIXING ELEMENT
ELEMENT D'ANCRAGE OSSEUX ET DISPOSITIF DE STABILISATION COMPRENANT UN TEL ELEMENT D'ANCRAGE OSSEUX

(30) Priorität: 07.11.2003 DE 10351978; 07.11.2003 US 518469 P; 21.11.2003 US 523946 P; 03.03.2004 US 550182 P
(43) Veröffentlichungstag der Anmeldung: 02.08.2006
(60) Teilanmeldung: 07012005.0
(73) Patentinhaber: BIEDERMANN MOTECH GmbH, 78054 VS-Schwenningen (DE)
(72) Erfinder: BIEDERMANN, Lutz, 78048 VS-Villingen (DE)
(74) Vertreter: Hofer, Dorothea
(86) Internationale Anmeldenummer: PCT/EP2004/012592
(87) Internationale Veröffentlichungsnummer: WO 2005/044123

(56) Entgegenhaltungen:
- WO-A-02/24087
- US-A- 5 074 865
- US-A1- 2002 055 737
- US-A1- 2002 055 740
- US-A1- 2002 055 742

## Beschreibung

Die Erfindung betrifft ein Knochenverankerungselement und eine Stabilisierungseinrichtung für Knochen bzw. für Wirbel mit einem derartigen Knochenverankerungselement.

Zur Fixierung von Knochenfrakturen oder zur Stabilisierung der Wirbelsäule sind Fixations- und Stabilisierungseinrichtungen bekannt, die aus wenigstens zwei im Knochen bzw. Wirbel verankerten und über eine Platte oder einen Stab verbundenen Knochenschrauben bestehen. Derartige starre Systeme erlauben keine Bewegung der relativ zueinander fixierten Knochenteile oder Wirbel.

Für bestimmte Indikationen ist jedoch eine dynamische Stabilisierung wünschenswert, bei der die zu stabilisierenden Knochenteile oder Wirbel eine kontrollierte begrenzte Bewegung zueinander ausführen können. Eine Realisierungsmöglichkeit für eine dynamische Stabilisierungseinrichtung besteht in der Verwendung eines beweglichen Elements anstelle eines die Knochenverankerungselemente verbindenden starren Stabes, wie es beispielsweise in der EP 0 669 109 B1 oder in der US 2003/0109880 A1 beschrieben ist.

Aus der US 5,474,555 ist ein Knochenverankerungselement in Form einer Polyaxial-Knochenschraube mit einem Schraubenelement und einem Aufnahmeteil zur Verbindung mit einem Stab bekannt, bei der das im Knochen zu verankernde Schraubenelement mit dem Aufnahmeteil derart verbunden ist, daß Schwenkbewegungen zwischen dem Schraubenelement und dem Aufnahmeteil möglich sind. Die beschriebene Lösung erlaubt jedoch keine Stabilisierung der Knochenteile untereinander mit einer kontrollierten Teilbewegung.

Aus der EP 1 273 269 ist eine Knochenschraube mit flexiblem Schaft bekannt. Die Schraube wird so eingesetzt, daß sie zwei Knochenteile oder zwei Knochen miteinander verbindet und als Zugelement dient. Durch den flexiblen Schaft wird eine Relativbewegung in Zugrichtung behindert, Bewegungen kleineren Umfangs in anderen Richtungen sind dagegen zugelassen. Werden zwei oder mehr Schrauben eingesetzt, so sind diese nicht miteinander verbunden. Eine ähnliche Schraube ist jeweils aus der DE 299 15 204 U1 und aus der US 4,959,064 bekannt.

Aus der US 2002/0055737 ist eine Vorrichtung zur Implantation in eine Wirbelsäule oder Hüfte bekannt. Sie umfasst eine Plattform mit einem helixförmigen Stachel, der einen Spitzenabschnitt, einen Verbindungsabschnitt und einen Zwischenteil aufweist.

Es ist Aufgabe der Erfindung ein Knochenverankerungselement und eine Stabilisierungseinrichtung für Knochen bzw. Wirbel mit einem solchen Knochenverankerungselement bereitzustellen, das bzw. die eine Stabilisierung von Knochen bzw. von Wirbeln mit einer kontrollierten Teilbewegung der zu stabilisierenden Knochenteile oder Wirbel erlaubt.

Die Aufgabe wird gelöst durch ein Knochenverankerungselement nach Patentanspruch 1 bzw. eine Stabilisierungseinrichtung nach Patentanspruch 12.

Weiterbildungen der Erfindung sind in den Unteransprüchen angegeben.

Die Erfindung weist den Vorteil auf, daß das Knochenverankerungselement aufgrund eines elastischen Abschnitts axiale Kräfte in Richtung seiner Schaftachse, Biegekräfte und Torsionskräfte aufnimmt. Damit wird eine durch die rücktreibende Kraft begrenzte Bewegung des Knochenteils oder Wirbels, in dem es verankert ist, erlaubt. Die elastischen Eigenschaften des Knochenverankerungselements können bei der Herstellung auf einfache Art durch Ändern der Dimensionen des elastischen Abschnitts verwirklicht werden. Das Knochenverankerungselement ist mit den bekannten Verbindungselementen wie Platten und Stäben verbindbar. Somit kann eine dynamische Stabilisierungseinrichtung mit einer gewünschten Bewegungsbegrenzung durch Auswahl von Knochenverankerungselementen mit geeigneten elastischen Eigenschaften unter Verwendung von herkömmlichen Platten oder Stäben bereitgestellt werden.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Schnittdarstellung einer ersten Ausführungsform des Knochenverankerungselements;
- Fig. 2: eine schematische Darstellung einer Stabilisierungseinrichtung nach einer ersten Ausführungsform, die das Knochenverankerungselement nach Fig. 1 aufweist;
- Fig. 3a: ein Seitenansicht des Knochenverankerungselements nach einer zweiten Ausführungsform mit einem eingelegten Stab;
- FIG. 3b: eine teilgeschnittene Ansicht des Knochenverankerungselements gemäß der Linie I-I von Fig. 3a;
- Fig. 3c: eine perspektivische Ansicht des Knochenverankerungselements von Fig. 3a ohne eingelegten Stab;
- Fig 4: eine teilgeschnittene Ansicht einer dritten Ausführungsform des Knochenverankerungselements;
- Fig. 5: eine schematische Ansicht einer Stabilisierungseinrichtung nach einer zweiten Ausführungsform, die das Knochenverankerungselement nach der dritten Ausführungsform verwendet;
- Fig. 6a: eine Explosionsdarstellung einer vierten Ausführungsform des Knochenverankerungselements;
- Fig. 6b: eine Schnittdarstellung eines Teils der Ausführungsform von Fig. 6a mit einer Weiterbildung;
- Fig. 6c: eine Schnittdarstellung des Teils von Fig. 6b entlang der Linie A-A;
- Fig. 7a: eine Seitenansicht des Knochenverankerungselements nach einer fünften Ausführungsform;
- Fig. 7b: eine Schnittansicht des Knochenverankerungselements nach Fig. 7a; und
- Fig. 8: eine schematische Darstellung einer dritten Ausführungsform der Stabilisierungseinrichtung.

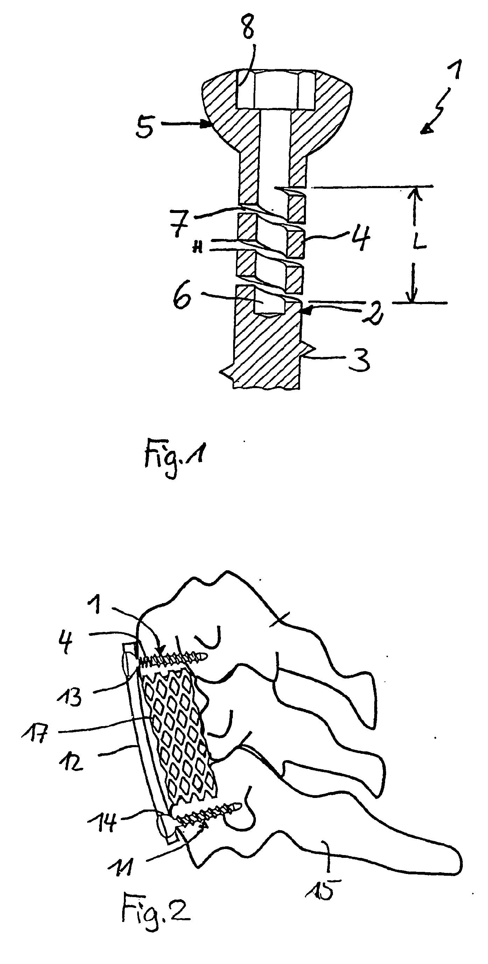

Die in Fig. 1 bis 3c und 7a-b gezeigten Ausführungsformen sind nicht Gegenstand der Erfindung und dienen lediglich der technischen Erläuterung.

Wie aus den Figuren 1 und 2 ersichtlich ist, ist das Knochenverankerungselement 1 in einer ersten Ausführungsform als einstückige Knochenschraube ausgebildet, die einen Schaft 2 mit einem ersten im Knochen zu verankernden Abschnitt 3 mit einem Knochengewinde und einen an den ersten Abschnitt 3 angrenzenden zweiten knochengewindefreien Abschnitt 4, sowie einen an den zweiten Abschnitt angrenzenden Kopf 5 aufweist. Ausgehend von dem freien Ende des Kopfs 5 erstreckt sich koaxial zu der Schraubenachse A eine Sackbohrung 6 mit vorbestimmten Durchmesser durch den Kopf 5 und den zweiten Abschnitt 4 hindurch. Im Bereich des zweiten Abschnitts 4 weist der Schaft 2 eine in Richtung der Schraubenachse spiralförmig mit einer vorbestimmten Steigung und über eine vorbestimmte Länge verlaufende, in radialer Richtung in die Bohrung 6 mündende Ausnehmung 7 auf. Dadurch ist der zweite Abschnitt 4 als elastisch in Form einer Schraubenfeder ausgebildet. Die Länge L des elastischen Abschnitts in Richtung der Schraubenachse, die Höhe H der Ausnehmung 7 in Richtung der Schraubenachse, die Steigung der Spirale und der Durchmesser der koaxialen Bohrung 6 sind so gewählt, daß eine gewünschte Steifigkeit der Schraubenfeder gegenüber axialen Kräften, Biegekräften und Torsionskräften, die auf die Knochenschraube wirken, gegeben ist.

Der Kopf 5 ist in dem gezeigten Ausführungsbeispiel linsenförmig ausgebildet und weist an seinem freien Ende eine sechskantförmige Ausnehmung 8 für einen Inbusschlüssel auf. Es ist jedoch auch eine andere Kopfform und/oder eine andere Ausnehmung, wie z.B. ein Kreuzschlitz, zum Eingreifen mit einem Einschraubwerkzeug möglich.

Eine Stabilisierungseinrichtung nach einer ersten Ausführungsform beinhaltet, wie in Fig. 2 gezeigt ist, ein erstes Knochenverankerungselement 1 in Form der Knochenschraube von Fig. 1 und ein zweites Knochenverankerungselement 11, das als herkömmliche Knochenschraube ohne elastischen Abschnitt ausgebildet sein kann, sowie eine Platte 12 mit Ausnehmungen 12, 13 zum Hindurchführen des Schafts der Knochenschrauben.

In dem in Fig. 2 dargestellten Anwendungsbeispiel ist die Stabilisierungseinrichtung zum Stabilisieren zweier Wirbel 15, 16, die nach Entfernung der Bandscheibe oder nach Entfernung eines dazwischenliegenden Wirbels über ein Fusionselement 17, z.B. einen Titanzylinder, miteinander steif verbunden werden sollen, vorgesehen.

Im Betrieb werden zuerst die Schäfte der Knochenschrauben 1, 11 durch die Ausnehmungen 12, 13 der Platte hindurchgeführt, und anschließend in den jeweiligen Wirbel 15, 16 eingeschraubt, bis die Platte 12 am Wirbel anliegt. Der elastische Abschnitt 4 der Knochenschraube 1 ist dabei im Wirbel versenkt. In diesem Fall ist eine limitierte Bewegung des Wirbels nur in Richtung der Schraubenachse ermöglicht. Wenn beim Einwachsen des Fusionselements ein Einsinken desselben in den Knochen stattfindet, gibt die Knochenschraube aufgrund des elastischen Abschnitts etwas nach. Damit wird verhindert, daß ungünstige Spannungen auftreten.

Bei der beschriebenen Stabilisierungseinrichtung können auch zwei oder mehr Knochenschrauben mit elastischem Abschnitt 4 verwendet werden. Bei mehr als zwei Schrauben weist die Platte eine entsprechende Anzahl von Ausnehmungen auf. Die Stabilisierungseinrichtung ist nicht nur für die Verwendung an der Wirbelsäule geeignet, sondern auch in anderen Fällen, in denen eine Plattenosteosynthese vorgenommen wird, anwendbar.

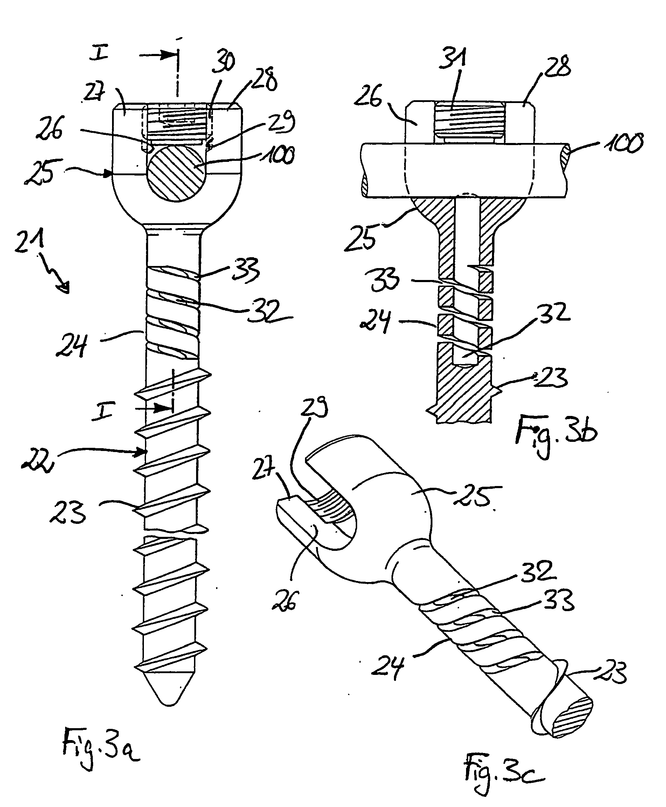

Ein Knochenverankerungselement 21 nach der in den Fig. 3a bis 3c gezeigten zweiten Ausführungsform ist als Monoaxial-Knochenschraube zum Verbinden mit einem Stab 100 ausgebildet. Die Monoaxial-Knochenschraube weist einen Schaft 22 mit einem ersten, im Knochen zu verankernden Abschnitt 23 mit einem Knochengewinde und einen an den ersten Abschnitt 23 angrenzenden knochengewindefreien Abschnitt 24 sowie ein mit dem Schaft 21 einstückig verbundenes Aufnahmeteil 25 zur Aufnahme des Stabes 100 auf. Das Aufnahmeteil 25 ist im wesentlichen zylindrisch ausgebildet und weist ausgehend von seinem freien Ende eine Ausnehmung 26 mit einem U-förmigen Querschnitt auf, die gerade so groß bemessen ist, daß der Stab 100 einlegbar ist und in den Grund der Ausnehmung 26 paßt. Durch die U-förmige Ausnehmung 26 sind zwei freie Schenkel 27, 28 gebildet, die angrenzend an ihr freies Ende ein Innengewinde 29 aufweisen, welches mit einem entsprechenden Außengewinde 30 einer zwischen die Schenkel einzuschraubenden Innenschraube 31 zum Fixieren des Stabs 100 zusammenwirkt.

Wie insbesondere aus Fig. 3b ersichtlich ist, erstreckt sich vom Grund der U-förmigen Ausnehmung 26 in Richtung zu dem Knochengewindeabschnitt 23 eine koaxiale Bohrung 32 mit einer vorbestimmten Tiefe durch den knochengewindefreien Abschnitt 24. Wie auch bei der ersten Ausführungsform weist der Schaft im Bereich des zweiten Abschnitts 24 eine spiralförmig in Richtung der Schraubenachse verlaufende Ausnehmung 33 auf, die in radialer Richtung in die Bohrung 32 mündet. Dadurch ist der zweite Abschnitt 24 elastisch und wirkt als Schraubenfeder.

Eine Stabilisierungseinrichtung nach einer zweiten Ausführungsform beinhaltet wenigstens ein Knochenverankerungselement, das als Monoaxialschraube mit dem elastischen Abschnitt 24 ausgebildet ist, ein zweites Knochenverankerungselement, das als herkömmliche Monoaxial-oder Polyaxial-Knochenschraube ohne einen elastischen Abschnitt ausgebildet ist, sowie einen Stab. Anstelle einer herkömmlichen Monoaxial- oder einer Polyaxial-Knochenschraube als zweites Verankerungselement kann die Stabilisierungseinrichtung auch zwei Monoaxial-Knochenschrauben gemäß Fig. 3a - 3c aufweisen.

Im Betrieb werden die Knochenverankerungselemente in den jeweiligen Knochen bzw. bei Anwendung an der Wirbelsäule in die jeweiligen Wirbel eingeschraubt, anschließend wird der Stab 100 in die Aufnahmeteile eingelegt und dann über die Innenschraube fixiert. Die Monoaxial-Knochenschraube 21 wird dabei soweit eingeschraubt, daß der elastische Abschnitt 24 über die Oberfläche des Knochens bzw. Wirbels wenigstens teilweise heraussteht. Bei einer Bewegung des Knochens bzw. Wirbels aus der zu stabilisierenden Ruhelage heraus wird über den elastischen Abschnitt 24 eine rücktreibende Kraft auf den Knochen bzw. Wirbel ausgeübt, die ihn wieder in die Ruhelage bringt und somit die Bewegung limitiert.

Alternativ kann die Knochenschraube soweit eingeschraubt werden, daß der elastische Abschnitt 24 nicht oder nur geringfügig über die Oberfläche des Knochens hervorsteht. In diesem Fall kann aufgrund der Federwirkung des elastischen Abschnitts ein Nachgeben nach der Justierung erfolgen.

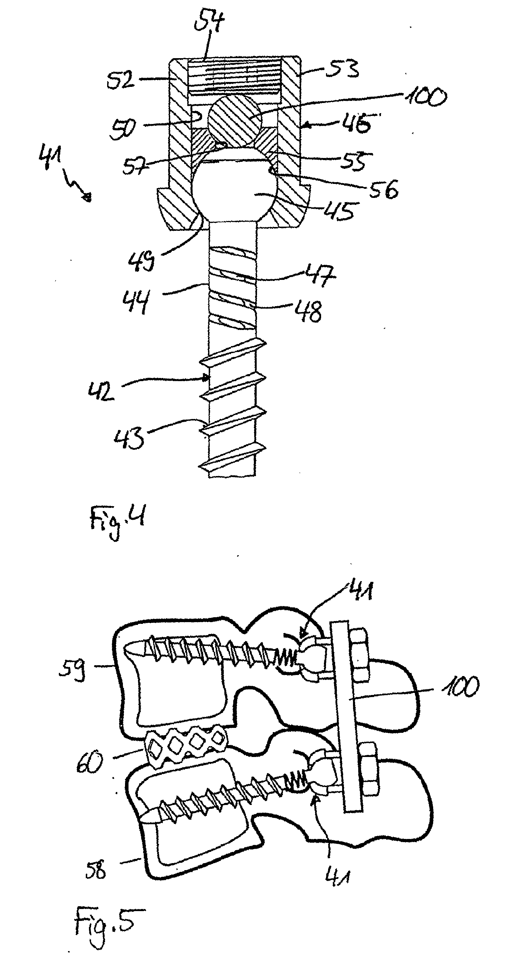

In einer vierten in Fig. 4 dargestellten Ausführungsform ist das Knochenverankerungselement 41 als Polyaxial-Knochenschraube ausgebildet. Diese weist ein einstückig ausgebildetes Schraubenelement mit einem Schaft 42 mit einem im Knochen zu verankernden ersten Abschnitt 43 und einem daran angrenzenden zweiten knochengewindefreien Abschnitt 44, sowie einem an den zweiten Abschnitt 44 angrenzenden kugelsegmentförmigen Kopf 45 auf. Der Kopf 45 ist in einem Aufnahmeteil 46 gehalten. Der zweite Abschnitt 44 ist wie bei den vorhergehenden Ausführungsformen als Schraubenfeder ausgebildet und weist eine koaxiale Bohrung 47, die sich vom freien Ende des Kopfs durch den zweiten Abschnitt hindurch erstreckt, und eine spiralförmig in der Wandung verlaufende Ausnehmung 48 auf.

Das Aufnahmeteil 46 ist im wesentlichen zylindrisch ausgebildet und weist an seinem einen Ende eine axialsymmetrisch ausgerichtete erste Bohrung 49 auf, deren Durchmesser größer als der des Schafts 42 und kleiner als der des Kopfs 45 ist. Ferner weist das Aufnahmeteil 46 eine koaxiale zweite Bohrung 50 auf, die auf dem der ersten 49 Bohrung gegenüberliegenden Ende offen ist und deren Durchmesser so groß ist, daß das Schraubenelement durch das offene Ende mit dem Schaft durch die erste Bohrung 45 hindurchführbar ist, bis der Kopf 45 am Rand der ersten Bohrung 49 anliegt. Das Aufnahmeteil 46 weist ebenso wie das Aufnahmeteil 25 der vorhergehenden Ausführungsform eine sich vom freien Ende in Richtung der ersten Bohrung 49 erstreckende U-förmige Ausnehmung auf, durch die zwei freie Schenkel 52, 53 gebildet sind. In einem Bereich angrenzend an ihr freies Ende weisen die Schenkel 52, 53 ein Innengewinde auf, welches mit einem entsprechenden Außengewinde einer Innenschraube 54 zum Fixieren des Stabs 100 zusammenwirkt.

Es ist ferner ein Druckelement 55 zum Fixieren des Kopfs in dem Aufnahmeteil vorgesehen, das so ausgebildet ist, daß es an seiner dem Kopf 45 zugewandten Seite eine sphärische Ausnehmung 56 aufweist, deren Radius im wesentlichen gleich dem Radius des kugelsegmentförmigen Abschnitts des Kopfes 45 ist. Der Außendurchmesser des Druckelements 55 ist so gewählt, daß das Druckelement in dem Aufnahmeteil 46 zu dem Kopf 45 hin verschiebbar ist. Das Druckelement weist ferner eine koaxiale Bohrung 57 für den Zugriff auf eine nicht dargestellte Ausnehmung in dem Schraubenkopf 45 zum Ineingriffbringen mit einem Einschraubwerkzeug auf.

Eine Stabilisierungseinrichtung nach einer dritten Ausführungsform umfaßt wenigstens zwei Knochenverankerungselemente und einen Stab, wobei wenigstens eines der Knochenverankerungselemente als Polyaxial-Knochenschraube mit einem Schaft mit einem elastischen Abschnitt 44 ausgebildet ist. Das zweite Knochenverankerungselement kann als herkömmliche Monoaxial-oder Polyaxial-Knochenschraube ohne den elastischen Abschnitt ausgebildet sein oder es kann als eine Monoaxial-Knochenschraube oder eine Polyaxial-Knochenschraube mit einem elastischen Abschnitt, wie beschrieben, ausgebildet sein.

Im Betrieb wird das Schraubenelement zuerst in das Aufnahmeteil eingeführt, bis der Kopf 45 am Rand der ersten Bohrung 49 anliegt. Dann wird das Schraubenelement in den Knochen bzw. Wirbel eingeschraubt, wobei der elastische Abschnitt 44 wenigstens teilweise über die Knochenoberfläche hervorsteht. Anschließend wird der Stab eingelegt, die Winkelstellung des Aufnahmeteils relativ zu dem Schraubenelement justiert und anschließend durch Festziehen der Innenschraube fixiert. Durch den elastischen Abschnitt werden wie bei der vorhergehenden Ausführungsform Bewegungen um die Ruhelage in begrenzter Weise ermöglicht.

Alternativ kann auch der Schaft soweit in den Knochen eingeschraubt werden, daß der elastische Abschnitt nicht oder nur geringfügig über die Knochenoberfläche hervorsteht.

Fig. 5 ist ein Anwendungsbeispiel der Stabilisierungseinrichtung nach der dritten Ausführungsform zum Stabilisieren zweier Wirbel 58, 59 die über ein Fusionselement 60, das eine entfernte Bandscheibe ersetzen soll, miteinander verbunden sind. Der Betrieb erfolgt wie zuvor beschrieben. Die begrenzte Bewegungsmöglichkeit der Wirbel 58, 59 zueinander führt zu einer erhöhten zyklischen Teilbelastung, wodurch das Knochenwachstum angeregt wird und die Verknöcherung schneller voranschreitet.

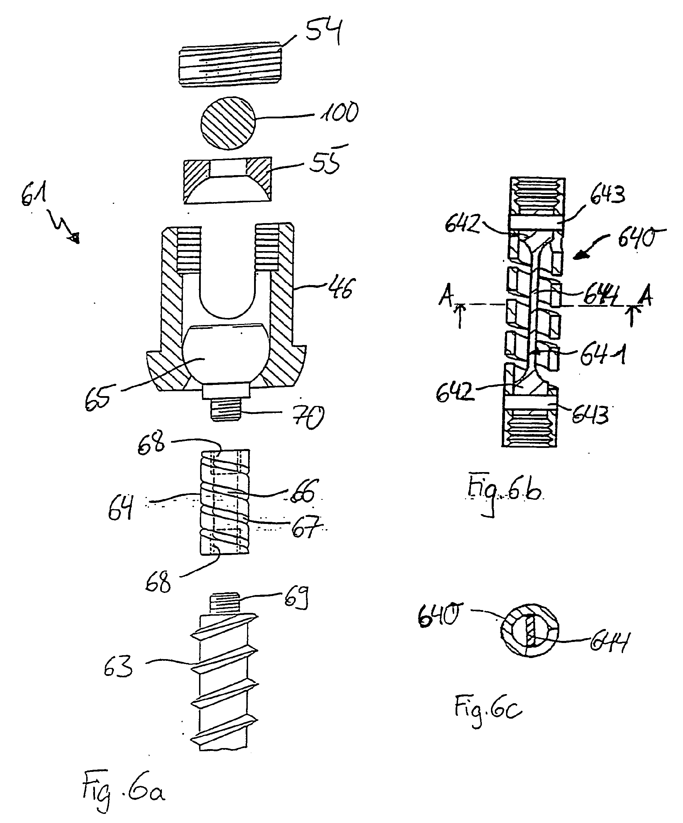

Die in Fig. 6a gezeigte vierte Ausführungsform des Knochenverankerungselements 61 unterscheidet sich von der in Fig. 4 gezeigten dritten Ausführungsform durch die Ausbildung des Schraubenelements. Alle anderen Bestandteile sind gleich zu der dritten Ausführungsform.

Bei dem Schraubenelement gemäß der vierten Ausführungsform sind der Knochengewindeabschnitt 63, der elastische Abschnitt 64 und der Kopf 65 als separate Teile ausgebildet. Der elastische Abschnitt 64 besteht aus einem zylindrischen Rohr mit einer durchgehenden koaxialen Bohrung 66 und einer spiralförmig in Richtung der Zylinderachse verlaufenden Ausnehmung 67 in der Wandung, die in radialer Richtung in die Bohrung 66 mündet. Dadurch ist eine Schraubenfeder wie bei den vorherigen Ausführungsformen gebildet. Angrenzend an sein jeweiliges freie Ende weist der elastische Abschnitt 64 ein sich über eine vorbestimmte Länge erstreckendes Innengewinde 68 an beiden Enden auf. Der Knochengewindeabschnitt 63 weist an seinem der einzuschraubenden Spitze gegenüberliegenden Ende einen zylindrischen Ansatz 69 mit einem Außengewinde auf, welches mit dem Innengewinde 68 des elastischen Abschnitts 64 zusammenwirkt.

Der Kopf 65 weist an seiner dem abgeflachten Ende gegenüberliegenden Seite einen zylindrischen Ansatz 70 mit einem Außengewinde, welches mit dem Innengewinde 68 des elastischen Abschnitts zusammenwirkt, auf.

Im Betrieb wird im Unterschied zur vorhergehenden Ausführungsform zuerst das Schraubenelement durch Zusammenschrauben des Knochengewindeabschnitts 63, des elastischen Abschnitts 64 und des Kopfs 65 zusammengesetzt und dann in das Aufnahmeteil eingeführt. Der weitere Betrieb erfolgt wie bei der vorhergehenden Ausführungsform.

Das Knochenverankerungselement nach dieser Ausführungsform hat den Vorteil, daß seine Herstellung vereinfacht ist. Ferner weist es den Vorteil auf, daß elastische Abschnitte 64 verschiedener Länge und verschiedener Steifigkeit bereitgehalten werden können und je nach Anwendung vor dem Einsatz ausgewählt werden und mit Köpfen einer Größe und Gewindeschäften vorbestimmter Länge zu einem Schraubenelement zusammengesetzt werden können.

Fig. 6b zeigt eine Weiterbildung des elastischen Abschnitts 64 aus Fig. 6a. Der elastische Abschnitt 640 gemäß Fig. 6b weist in seinem Inneren einen Kern 641 auf. Der Kern 641 ist an seinen Enden 642 zylindrisch ausgebildet mit einem Durchmesser, der so bemessen ist, daß der Kern in den Hohlraum des elastischen Abschnitts 640 einschiebbar ist. Die zylindrischen Abschnitte 642 und der elastische Abschnitt 640 weisen querverlaufende Bohrungen auf, in die Stifte 643 zum Befestigen des Kerns geschoben sind. Zwischen den zylindrischen Enden weist der Kern einen Abschnitt 644 mit im wesentlichen rechteckigem Querschnitt auf, wie insbesondere aus Fig. 6c ersichtlich ist. In einer Abwandlung hat der Kern einen anderen Querschnitt, wie z.B. oval oder asymmetrisch. Der Kern erlaubt die Einstellung der Biegesteifigkeit und/oder der Torsionssteifigkeit des elastischen Abschnitts. Die Steifigkeit des elastischen Abschnitts gegenüber einer Biegung in einer bestimmten Richtung hängt von der Orientierung des Kerns innerhalb des elastischen Abschnitts ab. Vorzugsweise ist der Kern aus einem Material mit geringerer Steifigkeit verglichen mit dem Material des elastischen Abschnitts ausgebildet. Die gezeigte Befestigung des Kerns ist nur beispielhaft dargestellt.

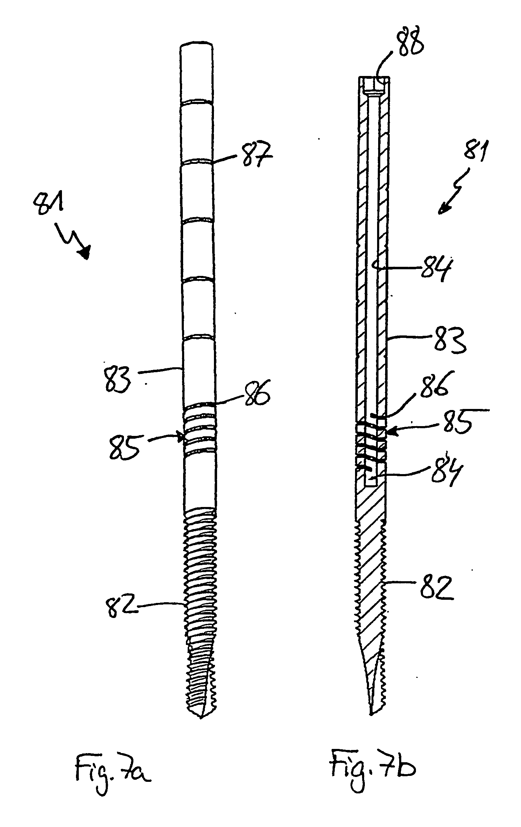

In einer weiteren, in den Fig. 7a und 7b gezeigten Ausführungsform ist das Knochenverankerungselement als Schanzschraube 81 ausgebildet. Die Schanzschraube 81 weist einen ersten Gewindeabschnitt 82 und einen sich daran anschließenden zylindrischen gewindefreien Schaftabschnitt 83 ohne Kopf auf, durch den sich eine vom freien Ende bis zum Gewindeabschnitt verlaufende koaxiale Sackbohrung 84 erstreckt. In einem vorbestimmten Bereich 85 des zylindrischen Abschnitts 83 ist eine spiralförmig sich in Richtung der Schraubenachse über eine vorbestimmte Länge erstreckende Ausnehmung 86 in der Wandung vorgesehen, durch wie bei den zuvor beschriebenen Ausführungsformen eine elastischer Abschnitt in Form einer Schraubenfeder gebildet ist. Ferner weist der zylindrische Schaftabschnitt 83 umfangsseitig in einem vorbestimmten Abstand voneinander angeordnete kreisförmige Einkerbungen 87 auf. Angrenzend an sein freies Ende weist der zylindrische Schaftabschnitt eine sechskantförmige Ausnehmung 88 oder eine beliebige andere Ausnehmung zum Eingreifen mit einem Einschraubwerkzeug auf.

Im Betrieb wird die Schanzschraube 81 insbesondere bei einem Fixateur externe zusammen mit herkömmlichen Schanzschrauben und herkömmlichen Verbindungselementen und Fixationsstäbe verwendet. Die Schanzschraube 81 wird in das zu fixierende Knochenfragment so eingeschraubt, daß der elastische Abschnitt 85 über die Knochenoberfläche wahlweise auch über die Hautoberfläche heraussteht. Je nach Steifigkeit des elastischen Abschnitts wird eine begrenzte Bewegung an vorbestimmter Stelle ermöglicht.

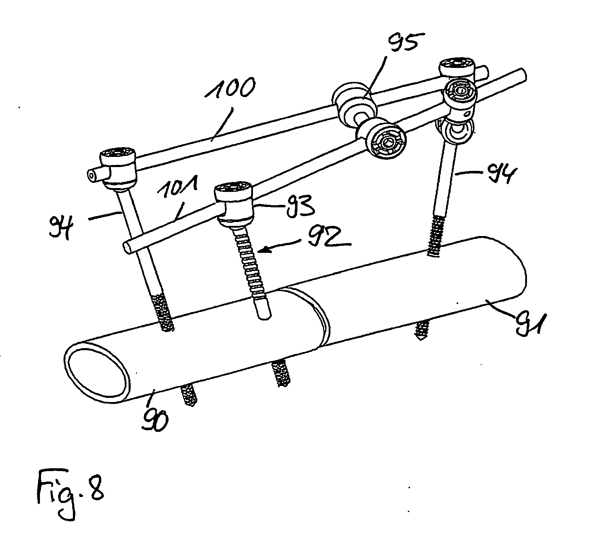

Fig. 8 zeigt eine Weiterbildung der Schanzschraube nach Fig. 7a und 7b in der Anwendung in einem Fixateur externe zur Stabilisierung von Röhrenknochenteilen 90, 91 eines gebrochenen Röhrenknochens. Die Schanzschraube 92 weist hierzu wie die Polyaxialschraube 41 der dritten Ausführungsform einen (nicht dargestellten) kugelsegmentförmigen Kopf auf, der in einem Aufnahmeteil 93 gehalten ist. Die Knochenteile 90, 91 werden über die Schanzschraube 92 und herkömmliche Polyaxial-Knochenschrauben 94, die über ein Verbindungselement 95 mit Stäben 100, 101 verbunden sind, stabilisiert.

Abwandlungen der vorher beschriebenen Ausführungsformen sind möglich. Insbesondere können Elemente einer Ausführungsform mit den Elementen einer anderen Ausführungsform kombiniert werden. Die mehrteilige Ausbildung des Schraubenelements gemäß der Ausführungsform nach Fig. 6a ist auch bei der Monoaxial-Knochenschraube nach den Fig. 3a bis 3c möglich, wobei dann das Aufnahmeteil in den elastischen Abschnitt einschraubbar ist. Ferner kann auch die Schanzschraube nach den Fig. 7a und 7b mehrteilig ausgebildet sein. In einer weiteren Abwandlung sind der Knochengewindeabschnitt und der elastische Abschnitt einstückig verbunden und nur der Kopf bzw. das Aufnahmeteil einschraubbar.

In einer weiteren Ausführungsform ist ein separater zylindrischer Kern vorgesehen, der in die Bohrung, die durch den elastischen Abschnitt hindurchgeht, eingeschoben wird. Dadurch läßt sich die Steifigkeit des elastischen Abschnitts zusätzlich einstellen. In einer weiteren Ausführungsform weist der elastische Abschnitt einen anderen Durchmesser auf als der Knochengewindeabschnitt. Bei einem größeren Durchmesser läßt sich so eine erhöhte Steifigkeit erzielen. Der Kern kann auch eine andere als eine zylindrische Form haben, beispielsweise kann er einen Abschnitt mit rechteckigem Querschnitt, wie in Fig. 6b und 6c dargestellt ist, aufweisen.

Die Erfindung ist nicht auf die konkret beschriebenen Beispiele der monoaxialen und der polyaxialen Knochenschraube beschränkt. Es können auch andere Ausbildungen derselben, insbesondere der Aufnahmeteile und der Fixiereinrichtungen möglich sein. Entscheidend ist, daß der Schaft einen elastischen Abschnitt aufweist. Ferner ist die Erfindung auch auf Haken anwendbar.

In einer weiteren Ausführungsform ist der elastische Abschnitt mit einem schlauchartigen Überzug versehen, der das Einwachsen von Gewebematerial oder Gefäßen verhindert.

Bei allen vorgenannten Ausführungsformen besteht der Vorteil, daß die begrenzte Bewegungsmöglichkeit der Knochenteile bzw. Wirbel zu einer erhöhten zyklischen Teilbelastung führt, die das Knochenwachstum anregt.

Die erfindungsgemäße Stabilisierungseinrichtung umfaßt wenigstens zwei Knochenverankerungselemente die jeweils mit einer externen Vorrichtung wie z.B. einer Platte oder einem Stab verbunden sind. Die Verbindung zwischen den Knochenverankerungselementen und der Platte oder dem Stab ist vorzugsweise fest. Die bei einer Bewegung der Knochen oder Knochenteile über die externe Vorrichtung auf den Kopf des Knochenverankerungselements einwirkenden Kräfte werden durch den elastischen Schaft von der Verankerung entkoppelt, so daß die Gefahr des Lockerwerdens der Verankerung im Knochen reduziert wird. Die Stabilisierungseinrichtung ist insbesondere zur Stabilisierung der Wirbelsäule anwendbar.

Als Materialien für das Knochenverankerungselement bzw. seine Bestandteile sind körperfreundliche Materialien einsetzbar. Beispielsweise werden körperfreundliche Metalle, wie z.B. Titan, oder ein körperfreundlicher Kunststoff eingesetzt. Auch die Verwendung einer Formgedächtnislegierung mit bekannten superelastischen Eigenschaften, z.B. Nitinol, ist möglich. Dabei kann das Knochenverankerungselement als ganzes aus einer solchen Legierung gebildet sein, einschließlich eines Kerns im elastischen Abschnitt, oder nur der Kern oder nur der elastische Abschnitt können daraus gebildet sein. Die Verwendung von verschiedenen Materialien für verschiedene Komponenten des Verankerungselements ist ebenso denkbar.

## Patentansprüche

1. Knochenverankerungselement mit einem Schaft (2; 22; 42; 63, 64; 82, 83) zum Verankern in einem Knochen, wobei der Schaft einen ersten Abschnitt (3; 23; 43; 63; 82) aufweist, der im Knochen verankerbar ist und einen zweiten Abschnitt, der elastisch ausgebildet ist, und mit einem Kopf,
**dadurch gekennzeichnet,**
**dass** der Kopf zum Verbinden mit einem wenigstens zwei Knochenverankerungselemente verbindenden Stab ausgebildet ist, wobei der Kopf und der Schaft gelenkig mit einander verbunden sind.

2. Knochenverankerungselement nach Anspruch 1, **dadurch gekennzeichnet, daß** der zweite Abschnitt (4; 24; 44; 64; 85) als Schraubenfeder ausgebildet ist.

3. Knochenverankerungselement nach Anspruch 1 oder 2, **dadurch gekennzeichnet, daß** der zweite Abschnitt eine zur Schaftachse koaxiale Bohrung (6; 32; 47; 66; 86) aufweist.

4. Knochenverankerungselement nach einem der Ansprüche 1 bis 3, **dadurch gekennzeichnet, daß** der zweite Abschnitt eine spiralförmig in Richtung der Schaftachse verlaufende Ausnehmung (7; 33; 48; 67; 86) in der Wandung des Schafts aufweist.

5. Knochenverankerungselement nach einem der Ansprüche 1 bis 4, **dadurch gekennzeichnet, daß** der Schaft (2; 22; 42; 82, 83) einstückig ausgebildet ist.

6. Knochenverankerungselement nach einem der Ansprüche 1 bis 4, **dadurch gekennzeichnet, daß** der zweite Abschnitt als separates Teil (64) ausgebildet ist.

7. Knochenverankerungselement nach einem der Ansprüche 1 bis 6, **dadurch gekennzeichnet, daß** der Kopf (65) zum Verbinden mit einer externen Vorrichtung als vom Schaft getrenntes Teil ausgebildet ist.

8. Knochenverankerungselement nach einem der Ansprüche 1 bis 7, **dadurch gekennzeichnet, daß** der zweite Abschnitt angrenzend an den Kopf vorgesehen ist.

9. Knochenverankerungselement nach einem der Ansprüche 1 bis 8, **dadurch gekennzeichnet, daß** das Knochenverankerungselement eine Knochenschraube (1; 21; 41; 61; 81) ist.

10. Knochenverankerungselement nach einem der Ansprüche 1 bis 9, **dadurch gekennzeichnet, daß** der Kopf im wesentlichen zylindrisch ausgebildet ist und an seinem dem Schaft abgewandten Ende eine U-förmige Ausnehmung zum Einlegen des Stabs aufweist.

11. Knochenverankerungselement nach einem der Ansprüche 1 bis 10, **dadurch gekennzeichnet, daß** die Winkelstellung des Kopfes relativ zu dem Schaft arretierbar ist.

12. Stabilisierungseinrichtung zum Stabilisieren von Knochen mit wenigstens zwei Knochenverankerungselementen und einer diese verbindenden externen Vorrichtung, wobei wenigstens ein Knochenverankerungselement einen Schaft mit einem elastischen Abschnitt nach einem der Ansprüche 1 bis 11 aufweist.

13. Stabilisierungseinrichtung nach Anspruch 12, **dadurch gekennzeichnet, daß** die externe Vorrichtung ein Stab ist.

14. Stabilisierungseinrichtung nach Anspruch 12, **dadurch gekennzeichnet, daß** die externe Vorrichtung eine Platte ist.

15. Stabilisierungseinrichtung nach Anspruch 13 oder 14, **dadurch gekennzeichnet, daß** die Knochenverankerungselemente fest mit der Platte oder dem Stab verbunden sind.

## Claims

1. Bone-anchoring element with a shaft (2; 22; 42; 63, 64; 82, 83) for anchoring in a bone, the shaft having a first portion (3; 23; 43; 64; 82) that can be anchored in the bone, and a second portion that is designed elastically, and with a head, **characterized in that** the head is designed for connection to a rod that connects at least two bone-anchoring elements, the head and the shaft being connected to each other in an articulated manner.

2. Bone-anchoring element according to Claim 1, **characterized in that** the second portion (4; 24; 44; 64; 85) is designed as a helical spring.

3. Bone-anchoring element according to Claim 1 or 2, **characterized in that** the second portion has a bore (6; 32; 47; 66; 86) coaxial with respect to the shaft axis.

4. Bone-anchoring element according to one of Claims 1 to 3, **characterized in that** the second portion has a recess (7; 33; 48; 67; 86) arranged in the wall of the shaft and extending in a spiral shape in the direction of the shaft axis.

5. Bone-anchoring element according to one of Claims 1 to 4, **characterized in that** the shaft (2; 22; 42; 82, 83) is designed in one piece.

6. Bone-anchoring element according to one of Claims 1 to 4, **characterized in that** the second portion is designed as a separate part (64).

7. Bone-anchoring element according to one of Claims 1 to 6, **characterized in that** the head (65), for connection to an external device, is designed as a part separate from the shaft.

8. Bone-anchoring element according to one of Claims 1 to 7, **characterized in that** the second portion is provided adjoining the head.

9. Bone-anchoring element according to one of Claims 1 to 8, **characterized in that** the bone-anchoring element is a bone screw (1; 21; 41; 61; 81).

10. Bone-anchoring element according to one of Claims 1 to 9, **characterized in that** the head has a substantially cylindrical design and, at its end directed away from the shaft, has a U-shaped recess for insertion of the rod.

11. Bone-anchoring element according to one of Claims 1 to 10, **characterized in that** the angle setting of the head relative to the shaft can be locked.

12. Stabilizing appliance for stabilization of bone, with at least two bone-anchoring elements and with an external device connecting these, at least one bone-anchoring element having a shaft with an elastic portion according to one of Claims 1 to 11.

13. Stabilizing appliance according to Claim 12, **characterized in that** the external device is a rod.

14. Stabilizing appliance according to Claim 12, **characterized in that** the external device is a plate.

15. Stabilizing appliance according to Claim 13 or 14, **characterized in that** the bone-anchoring elements are connected fixedly to the plate or to the rod.

## Revendications

1. Élément d'ancrage osseux avec une tige (2 ; 22 ; 42 ; 63 ; 64 ; 82 ; 83) pour ancrage dans un os, la tige présentant un premier tronçon (3 ; 23 ; 43 ; 63 ; 82), susceptible d'être ancré dans l'os, et un deuxième tronçon de réalisation électrique, et avec une tête, **caractérisé en ce que** la tête est réalisée pour liaison à une barre reliant au moins deux éléments d'ancrage osseux, la tête et la tige étant reliées ensemble de façon articulée.

2. Élément d'ancrage osseux selon la revendication 1, **caractérisé en ce que** le deuxième tronçon (4 ; 24 ; 44 ; 64 ; 85) est réalisé sous la forme de ressort hélicoïdal.

3. Élément d'ancrage osseux selon la revendication 1 ou 2, **caractérisé en ce que** le deuxième tronçon présente un perçage (6 ; 32 ; 47 ; 66 ; 86) coaxial par rapport à l'axe de tige.

4. Élément d'ancrage osseux selon l'une des revendications 1 à 3, **caractérisé en ce que** le deuxième tronçon présente, dans la paroi de la tige, un évidement (7 ; 33 ; 48 ; 67 ; 86) s'étendant en forme de spirale dans la direction de l'axe de tige.

5. Élément d'ancrage osseux selon l'une des revendications 1 à 4, **caractérisé en ce que** la tige (2; 22 ; 42 ; 82 ; 83) est réalisée d'une seule pièce.

6. Élément d'ancrage osseux selon l'une des revendications 1 à 4, **caractérisé en ce que** le deuxième tronçon est réalisé sous la forme de partie (64) séparée.

7. Élément d'ancrage osseux selon l'une des revendications 1 à 6, **caractérisé en ce que** la tête (65) est réalisée, pour la liaison à un dispositif externe, réalisé sous forme de partie séparée de la tige.

8. Élément d'ancrage osseux selon l'une des revendications 1 à 7, **caractérisé en ce que** le deuxième tronçon est prévu limitrophe à la tête.

9. Élément d'ancrage osseux selon l'une des revendications 1 à 8, **caractérisé en ce que** l'élément d'ancrage osseux est une vis à os (1 ; 21 ; 41 ; 61 ; 81).

10. Élément d'ancrage osseux selon l'une des revendications 1 à 9, **caractérisé en ce que** la tête est de forme sensiblement cylindrique et présente, à son extrémité opposée à la tige, un évidement en forme de U, pour insertion de la barre.

11. Élément d'ancrage osseux selon l'une des revendications 1 à 10, **caractérisé en ce que** la position angulaire de la tête par rapport à la tige est susceptible d'être bloquée.

12. Dispositif de stabilisation pour stabiliser de l'os, avec au moins deux éléments d'ancrage osseux, et un dispositif externe reliant ceux-ci, sachant qu'au moins un élément d'ancrage osseux présente une tige selon l'une des revendications 1 à 11, avec un tronçon élastique.

13. Dispositif de stabilisation selon la revendication 12, **caractérisé en ce que** le dispositif externe est une barre.

14. Dispositif de stabilisation selon la revendication 12, **caractérisé en ce que** le dispositif externe est une plaque.

15. Dispositif de stabilisation selon la revendication 13 ou 14, **caractérisé en ce que** les éléments d'ancrage osseux sont reliés rigidement à la plaque ou à la barre.
